# EUROPEAN PATENT SPECIFICATION

(11) **EP 2 948 128 B1**
(45) Date of publication and mention of the grant of the patent: **05.10.2016**
(21) Application number: 14700180.4
(22) Date of filing: 09.01.2014
(51) Int. Cl.: A61Q 19/08, A61K 8/49, A61K 8/67, A61K 8/97

(54) **SKIN CARE COMPOSITION**
HAUTPFLEGEZUSAMMENSETZUNG
COMPOSITION DE SOINS DE PEAU

(30) Priority: 28.01.2013 EP 13152808
(43) Date of publication of application: 02.12.2015
(73) Proprietor: Unilever N.V., 3013 AL Rotterdam (NL); Unilever PLC, London, Greater London EC4Y 0DY (GB)
(72) Inventor: DAMODARAN, Anita, Bangalore 560 066 (IN); DIAS, Paul Mark, Bangalore 560 066 (IN)
(74) Representative: Corsten, Michael Allan
(86) International application number: PCT/EP2014/050269
(87) International publication number: WO 2014/114496

(56) References cited:
- WO-A2-2008/037740
- FR-A1- 2 883 171
- US-A1- 2006 148 727
- US-A1- 2009 061 023
- E. S. G. STROES ET AL: "Folic Acid Reverts Dysfunction of Endothelial Nitric Oxide Synthase", CIRCULATION RESEARCH, vol. 86, no. 11, 9 June 2000 (2000-06-09), pages 1129-1134, XP055068977, ISSN: 0009-7330, DOI: 10.1161/01.RES.86.11.1129
- U. HEINRICH ET AL: "Green Tea Polyphenols Provide Photoprotection, Increase Microcirculation, and Modulate Skin Properties of Women", THE JOURNAL OF NUTRITION, vol. 141, no. 6, 27 April 2011 (2011-04-27), pages 1202-1208, XP055068990, ISSN: 0022-3166, DOI: 10.3945/jn.110.136465
- ANOYMOUS: 'Formulating anti-ageing products with folic acid' PERSONAL CARE, INGREDIENTS, FORMULATION, MANUFACTURE vol. 3, no. 1, 01 July 2009, XP055259648
- DAL BELO SE ET AL: 'Skin penetration of epigallocatechin-3-gallate and quercetin from green tea and Ginkgo biloba extracts vehiculated in cosmetic formulations. - PubMed - NCBI' SKIN PHARMACOL PHYSIOL vol. 22, 25 September 2009, pages 299 - 304, XP055259654

## Description

### Field of the Invention

The invention relates to a topical composition that provides enhanced skin health by way of providing anti-aging benefits that are evident from visibly reduced wrinkles and under-eye dark circles.

### Background of invention

Life-style modification and age increases the probability to acquire major health problems like cardiovascular risk factors. Impairment in blood vessel flexibility is a major risk factor for the onset of such diseases, reflected as hypertension, angina and atherosclerosis. In humans, the investigation of endothelial function has centered on either the macrocirculation or the microvasculature. Microcirculation is the delivery of fresh blood to the smallest blood vessels, present in the vasculature embedded within organ tissues. This contrasts with macrocirculation, which transport blood to and from the organs. The main functions of the microcirculation include 1. regulation of blood flow and tissue perfusion 2. regulation of blood pressure, 3. regulation of tissue fluid (swelling or edema), 4. delivery of oxygen and other nutrients and removal of carbon dioxide and other metabolic waste products, 5. regulation of body temperature, and 6. reduction of sedentary aging (wrinkles, under eye dark circles). In addition to maintaining these functions for overall health benefits, microcirculation enhances skin health.

Skin microcirculation is a complex and dynamic system which is important for thermoregulation, skin metabolism and trans-cutaneous penetration. The blood supply to the skin is provided by a network of arterioles, capillaries and venules organized into a superficial and a deep plexus. Skin is exposed to environmental stressors (such as UV, chemical pollutants and particulate matter) or physiological (non-environmental) stressors (psychological stress, sedentary aging and inflammation). Such compromised blood flow leads to physiological effects such as under-eye dark circles, wrinkles, retarded wound healing and edema.

To overcome these issues, for better skin health, the present inventors have approached the problem by combining actives that regulate macro vascular and micro vascular endothelial function. They have found that a composition comprising gallated catechins and folic acid in specific ratios is able to interact synergistically to enhance skin health when applied topically.

US2009061023 discloses a nutritional supplement for inhibiting sensorineural hearing loss which includes several micronutrients selected from folic acid, green tea extract and several others like thiamin, hydroxycobalamin, magnesium, zinc, selenium, and manganese to name a few. The claimed benefit is believed to be effected by improved microcirculation among others.

US2005106263 discloses a natural formulation for treatment of male, female and adolescent pattern hair loss comprising a combination of green tea leaf extract, polyphenols, epigallocatechin gallate (EGCG), vitamin E, folic acid, copper (as amino acid chelate), vitamin B12, zinc (as oxide), calcium pantothenate, niacin, biotin, riboflavin, thiamine, and optionally inositol, black tea extract and nettle extract.

The compositions disclosed in the above mentioned published documents do not disclose the specific ratios of gallated catechins and folic acid which the present inventors have determined to interact synergistically to improve micro and macro circulation for enhanced skin health.

It is thus an object of the present invention to provide for a topical composition that exhibits enhanced micro and/or macro-circulation for improved skin health.

### Summary of the Invention

According to the first aspect of the invention there is provided a topical composition comprising
(i) 0.1 to 5 wt% folic acid;
(ii) 0.1 to 5 wt% a gallated catechin; and
(iii) a cosmetically acceptable base selected from the group consisting of cream, lotion, gel and emulsion.
wherein the weight ratio of gallated catechin: folic acid is in the range of 1:1 to 10:1.

According to yet another aspect of the present invention there is provided a method of improving microcirculation of skin comprising applying to the skin a composition of the invention.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

"Topical composition" as used herein, is meant to include a composition for application to the external surface e.g. skin of mammals, especially humans for better skin health benefits. Such a composition may be generally classified as leave-on or rinse off, preferably leave-on and includes any product applied to a human body primarily for enhanced health of skin but may be used also for improving appearance, cleansing, odour control or general aesthetics. The composition of the present invention can be in the form of a liquid, lotion, cream, foam, scrub, gel, soap bar or toner, or applied with an implement or via a face mask, pad or patch. Non-limiting examples of such topical compositions include leave-on skin lotions and creams, antiperspirants, deodorants, lipsticks, foundations, mascara, sunless tanners or sunscreen lotions, and wash-off products like shampoos, conditioners, shower gels, or toilet bars,. "Skin" as used herein is meant to include skin on the face and body (e.g., neck, chest, back, arms, underarms, hands, legs, buttocks and scalp) and especially to the under-eye portions of the face. The topical composition of the invention is especially useful for application on skin areas that get wrinkled or are more likely to get wrinkled especially to the sun exposed parts of the body.

The first aspect of the present invention provides for a topical composition comprising folic acid and a gallated catechin in a cosmetically acceptable base
wherein the weight ratio of gallated catechin: folic acid is in the range of 1:1 to 10:1.

Folic acid is an important micronutrient. Folic acid has the structure as given below:

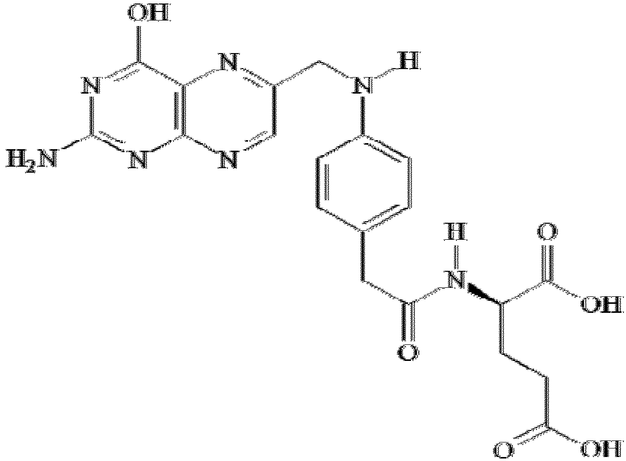

Folic acid (also known as folate, vitamin M, vitamin B9, vitamin Bc (or folacin), pteroyl-L-glutamic acid, pteroyl-L-glutamate, and pteroylmonoglutamic acid) are forms of the water-soluble vitamin B9. Folic acid is itself not biologically active, but its biological importance is due to tetrahydrofolate and other derivatives after its conversion to dihydrofolic acid in the liver.

Vitamin B9 (folic acid and folate) is essential to numerous bodily functions. The human body needs folate to synthesize DNA, repair DNA, and methylate DNA as well as to act as a cofactor in certain biological reactions. It is especially important in aiding rapid cell division and growth, such as in infancy and pregnancy. Children and adults both require folic acid to produce healthy red blood cells and prevent anemia.

Leafy vegetables are principal sources of folic acid, although in Western diets fortified cereals and bread may be a larger dietary source.

Foods that are high in folate include:
- Fruits particularly kiwi fruit and papaya.
- Vegetables such as broccoli, brussel sprouts, spinach, cabbage, asparagus and parsnips.
- Cooked kidney and liver
- Oranges (including orange juice)
- Tinned baked beans
- Lettuce, peas and cauliflower
- Egg yolks
- Milk

Folic acid is present in 0.1 to 5% preferably in 0.1 to 3%, more preferably 0.1 to 2% in the composition of the invention.

Gallated catechins are present in the composition of the invention. Gallated catechins are preferably epigallo catechin gallate (EGCG) or epicatechin gallate (ECG) or catechin gallate (CG). EGCG and ECG have the structure as given below:

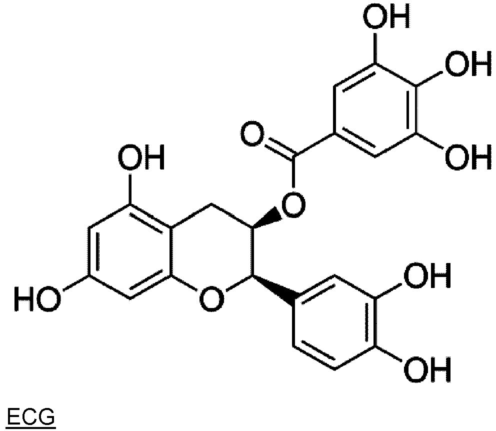

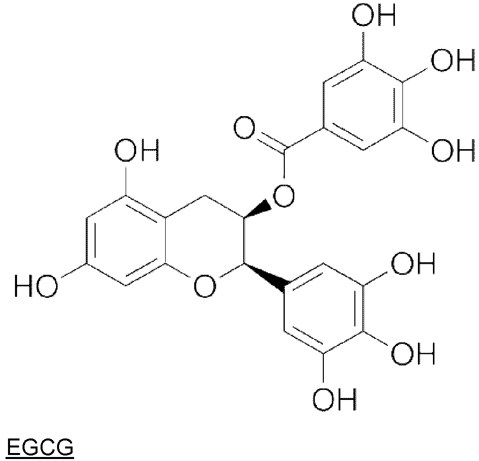

These catachins are found in high amounts in green tea from which they are preferably extracted. Green tea is made from the tea plant. Tea refers to one or more plants belonging to the family of *Camellia sinensis var. sinensis* and/or *Camellia sinensis var. assamica.* Tea is the second most consumed beverage worldwide. It is rich source of monomeric and polymeric forms of the flavonoids and can account up to 10-30 % flavonoids by weight.

Green tea is generally prepared from the leaves and buds of the tea plant by the process described below.

Tea (Camellia sinensis) is the second most consumed beverage worldwide. At harvest tea leaves contain high levels of catechins, a particular class of polyphenols. After harvest catechins may be rapidly converted by enzymatic oxidation to a complex mixture of other derivatives, thearubigins and theaflavins, responsible for the characteristic color of oolong and black tea. Green tea (GT), however, is produced by heat-treating leaves soon after harvest, thereby preserving the catechins from oxidation. The amount of catechins in a cup of GT is highly variable, depending on the precise type of tea, the ratio of dry tea to water and on the time that the leaves are infused before consumption. An average serving of 250 ml of GT contains between 50 and 100 mg of catechins. Catechins are the main bioactive constituents of green tea leaves and account for 25% to 35% of their dry weight. The polyphenolic flavonoid-type catechin are (+)-catechin (C), (-)-epicatechin (EC), (+)-gallocatechin (GC), (-)-epigallocatechin (EGC), (+)-catechin gallate (CG), (-)-epicatechin. gallate (ECG), (+)-gallocatechin gallate (GCG) and (-)-epigallocatechin gallate (EGCG). Catechins are also found in many commonly consumed fruits and beverages like apples, black, red and white currants, blueberries, chocolates, cocoa, grape seeds, and red wine.

The principle component in green tea is mentioned in the table below

**Table 1 - Principle components of green tea**

| | Green Tea (% weight of extract solids) |
|---|---|
| Catechins | 30-42 |
| Flavonols | 5-10 |
| Other flavonoids | 2-4 |
| Theogallin | 2-3 |
| Other depsides | 1 |
| Ascorbic Acid | 1-2 |
| Gallic Acid | 0.5 |
| Quinic acid | 2 |
| Other organic acids | 4-5 |
| Theanine | 4-6 |
| Other amino acids | 4-6 |
| Methylxanthines | 7-9 |
| Carbohydrates | 10-15 |
| Minerals | 6-8 |
| Volatiles | 0.02 |

Amongst the catechins the composition of different catechins in green tea are as follows.

**Flavonoid composition of Green tea: percent by dry weight.**

| Component | Gree tea |
|---|---|
| TotalFlavonoids | 15-25% |
| Total Catechins | 12-18% |
| (-) Epicatechin | 1-3% |
| (-) Epicatechingallate | 3-6% |
| (-) Epigallocatechin | 3-6% |
| (-) Epigallocatechingallate | 9-13% |
| Flavonols | 2-3% |
| Theaflavins | <1% |
| Other polyphenols | 2-4% |

Gallated catechins are present in 0.1 to 5%, preferably 0.1 to 4%, more preferably 0.1 to 3% in the composition of the invention.

The gallated catechins and folic acid are included in the composition of the invention in a weight ratio of gallated catechin: folic acid is in the range of 1:1 to 10:1, preferably in range of 2.5:1 to 7.5:1.

Without wishing to be bound by theory it is believed that the problem of poor blood circulation leading to the problems of poor skin health like under-eye dark circles, wrinkles etc occur due to the mechanism described below. Further, described below is the mechanism by which the composition of the present invention is believed to work to alleviate the problem.

Skin is exposed to environmental stressors (such as UV, chemical pollutants and particulate matter) or physiological (non-environmental) stressors (psychological stress, sedentary aging and inflammation), which triggers sequential changes; thereby induce significant changes in cutaneous blood flow due to endothelial dysfunction or nitric oxide (NO) impairment. Such compromised blood flow leads to physiological effects such as under-eye dark circles, wrinkles, retarded wound healing, and edema. All these physiological functions are linked with chronic low grade inflammation and peripheral resistance (endothelial dysfunction).

The present inventors believe that this compromised state of endothelium can be reverted to gain its normal functionality, by modulating endothelial NO production, either by interfering with NO signaling or by scavenging NO dissipating free radicals.

The mechanism underlying this effect is activation of endothelial nitric oxide synthase (eNOS), which results in enhanced nitric oxide which will result in vessel dialation. EGCG, a major gallated-catechin in green tea is believed to act as pro-oxidant at higher concentrations, while its presence at lower concentrations confers antioxidant effect in different cell types. EGCG exposure to endothelial cells, invitro potentiates eNOS activation, while presence of high concentration of EGCG leads to activation of pro-oxidant milieu. The pro-oxidant milieu is characterized by increased production of superoxide. This increased superoxide production may be due to conformational change in the enzyme called as eNOS 'uncoupling'. This enzyme is a homo-dimer under normal physiology and produces nitric oxide with stimulation (which is benefitial), and alters into its momomeric form under pro-oxidant milieu. The monomeric form produces superoxide instead of nitric oxide. The superoxide formed even scavenges minimal amount of NO in the surrounding to form peroxinitrites. Peroxinitrites are believed to cause protein nitrosylation and hamper normal signaling.

Uncoupling of eNOS is a major cause of endothelial dysfunction in both micro and macro vascular pathophysiology. At molecular level, administration of folic acid, a precursor of methyl tetrahydro folate (MTHF, co-factor to eNOS) keeps the enzyme in its coupled state (the active form). Thus folic acid pretreatment reduces superoxide production and enhances NO synthesis by the enzyme.

The present inventors have found that, when EGCG is treated in endothelial cells, it activated eNOS enzyme, but at higher concentration lead to superoxide formation. Pretreatment with folic acid abolished eNOS uncoupling, hence enhances EGCG induced NO production. Thus, a carefully selected combination of gallated catechins and folic acid produces the desired benefits which are not evident outside the selected concentration ratios.

The composition of the invention comprises a cosmetically acceptable base. The cosmetically acceptable base as per the present invention is a cream, lotion, gel or emulsion. The cosmetically acceptable base preferably comprises a fatty acid or a silicone compound. When the cosmetically acceptable base comprises fatty acid it is preferably present in 1 to 25% by weight of the composition. When the cosmetically acceptable bases are such as to have a product in a cream, lotion, or emulsion format, it generally comprises fatty acid. Of these formats, a more preferred format is a cream or lotion, further more preferably a cream. Vanishing cream base is one which comprises 3 to 25%, more preferably 5 to 20% fatty acid, which is a preferred format of the composition of the invention. In this, the base preferably comprises 0.1 to 10%, more preferably 0.1 to 3% soap. C₁₂ to C₂₀ fatty acids are especially preferred in vanishing cream bases, further more preferred being C₁₄ to C₁₈ fatty acids. In creams, the fatty acid is preferably substantially a mixture of stearic acid and palmitic acid. Soaps in the vanishing cream base include alkali metal salt of fatty acids, like sodium or potassium salts The soap is preferably the potassium salt of the fatty acid mixture. The fatty acid in vanishing cream base is often prepared using hystric acid which is substantially (generally about 90 to 95%) a mixture of stearic acid and palmitic acid (usually 55% stearic acid and 45% palmitic acid). Thus, inclusion of hystric acid and its soap to prepare the vanishing cream base is within the scope of the present invention. It is particularly preferred that the composition comprises at least 6%, preferably at least 10%, more preferably at least 12% fatty acid. The cosmetically acceptable base is usually from 10 to 99.9%, preferably from 50 to 99% by weight of the composition. Another preferred base is a lotion. Lotions generally comprise 1 to 20% fatty acid. The cosmetically acceptable base preferably includes water. Water is preferably included in 35 to 90%, more preferably 50 to 85%, further more preferably 50 to 80% by weight of the composition.

An especially suitable cosmetically acceptable base is one which comprises a water-in-oil emulsion comprising silicone oils as the continuous phase. The water in oil emulsions preferably comprise a crosslinked silicone elastomer blend.

Inclusion of silicone elastomer blend in a water-in-oil emulsion may be used as the cosmetically acceptable base for preparing the compositions of the present invention. While silicone fluids may be used, silicone elastomers which are cross-linked, are especially preferred. The creation of cross-linkages between linear polymers, such as dimethicone, converts the linear polymer into a silicone elastomer. In contrast to silicone fluid polymers, the physical properties of elastomers are typically dependent on the number of cross-linkages, rather than molecular weight. The ability of silicone elastomers to swell makes them ideal thickeners for oil phases. The elastomers have a very smooth and soft feel when applied to skin or hair. They can also be used as delivery agents for fragrances, vitamins and other additives in cosmetic compositions.

Suitable silicone elastomer blends or gels which are commercially available and suitable for inclusion in the composition of the invention and found to provide the enhanced stability are: Dow Corning® EL-8051 IN Silicone Organic Elastomer Blend [INCI Name: Isodecyl Neopentanoate (and) Dimethicone/Bis Isobutyl PPG-20 Crosspolymer]; EL-8050 [INCI Name: Isododecane (and) Dimethicone/Bis-Isobutyl PPG 20 Crosspolymer] DC 9040, DC9041, DC9045 (Dimethicone crosspolymer); DC 9506, 9509 (Dimethicone vinyl dimethicone crosspolymer); Shin-Etsu KSG-15, KSG-16, KSG-17 (Dimethicone vinyl dimethicone crosspolymer). It is further preferred that the composition comprises 5 to 50% silicone elastomer by weight of the composition.

Useful sun-protective agents e.g. inorganic sun-blocks may be preferably used in the present invention. These include, for example, zinc oxide, iron oxide, silica, such as fumed silica, or titanium dioxide. The total amount of sun block that is preferably incorporated in the composition according to the invention is from 0.1 to 5% by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided a method of improving microcirculation of skin comprising applying to the skin a composition of the invention. The use of such a method is preferably non-therapeutic.

According to another aspect the invention provides a composition for use in improving microcirculation of skin.

The invention will now be illustrated with the help of the following non-limiting examples.

### Examples

The experiments were conducted using the following procedure:

### Materials

DAF FM-DA was purchased from Invitrogen (Eugene, Oregon)
DMEM, Folic acid (Cat # - F7876) and EGCG (Cat #- 50299) were purchased from Sigma (St. Louis, MO, USA).

### Preparation of EA.Hy926 cells

The cultured human endothelial cell line EA.Hy926 cells were procured from American Type Culture Collection (CRL-2922 - ATCC) and were cultured in DMEM (Sigma) supplemented with 2mM L-glutamine, 100U/ml penicillin, 100µg/ml streptomycin and 10% vol/vol FBS (Gibco, Invitrogen). Cells were cultured at 37°C in 95% humidified air with 5% CO₂. After attaining 70-80% confluence, the cells were sub-cultured by trypsinization (0.25% Tryp-EDTA, for 2-3 min). For experimental purpose, cells were seeded onto 24 well tissue culture plates (CLS3524 Sigma). After adherence, cells were subjected to starvation in serum free low glucose DMEM for 12-14 hrs prior to any experiment, to maintain the cells in quiescent state and reduce the basal level of NO production.

### Measurement of intracellular nitric oxide using flow-cytometry

### Experimental flow:

1. EAHy926 (5x10⁵) cells were seed into 24 well tissue culture plate and allowed to adhere for 12-16 hrs.
2. After adherence, cells were treated with or without 4.41 µg/ml (10µM) folic acid and incubated for 24 hrs in DMEM without FBS.
3. The cells in 24 well tissue culture plates were loaded with DAF FM-DA (1µM) for 30 min, washed twice with serum free medium.
4. Subsequently, cells were stimulated with different concentration of EGCG for 30 mins and washed twice with serum free medium.
5. The stimulated cells were trypsinized with 0.25% Trypsin-EDTA and fixed with 2% PFA for 15 min.
6. The suspension of cells were acquired using flow-cytometry. A population of 10,000 cells were gated and segregated based on their relative fluorescence intensities using FACS Calibur (BD; SanDiego). The mean yield of two distinct populations was measured and compared with the respective population in untreated cells.

The data on the Nitric Oxide activity is summarized in Table -1 for the various compositions which may be gallated catechins (EGCG) alone at various concentrations or folic acid alone or EGCG in combination with folic acid. Compositions exhibiting an NO activity value higher than 3.0 preferably higher than 3.8 are taken as those expected to provide the enhanced microcirculation benefits of the present invention.

**Table - 1**

| Example Number | Concentration of EGCG, µg | Concentration of Folic acid, µg | Ratio of EGCG: Folic acid | NO activity |
|---|---|---|---|---|
| 1 | 0 | 0 | - | 1.00 |
| 2 | 2.29 | 0 | - | 2.87 |
| 3 | 4.58 | 0 | - | 2.98 |
| 4 | 11.45 | 0 | - | 3.75 |
| 5 | 22.91 | 0 | - | 3.58 |
| 6 | 33.07 | 0 | - | 2.32 |
| 7 | 44.1 | 0 | - | 1.64 |
| 8 | 110.25 | 0 | - | 0.63 |
| 9 | 0 | 4.41 | 0 | 1.00 |
| 10 | 2.29 | 4.41 | 0.52 | 2.73 |
| 11 | 4.58 | 4.41 | 1.04 | 3.27 |
| 12 | 11.45 | 4.41 | 2.60 | 4.37 |
| 13 | 22.91 | 4.41 | 5.20 | 5.59 |
| 14 | 33.07 | 4.41 | 7.50 | 3.96 |
| 15 | 44.1 | 4.41 | 10.0 | 3.50 |
| 16 | 110.25 | 4.41 | 25.0 | 3.09 |
| 17 | 220.5 | 4.41 | 50.0 | 2.29 |
| 18 | 0 | 1.00 | - | 1.08 |
| 19 | 0 | 5.00 | - | 1.02 |
| 20 | 0 | 10.00 | - | 1.03 |
| 21 | 0 | 25.00 | - | 1.03 |

The data in Table - 1 indicates that increasing folic acid concentration alone does not improve the NO activity. Increasing the EGCG concentration alone increases NO activity till an EGCG concentration of about 11 µg and thereafter the NO activity decreases. Further, it is to be noted that an NO activity of 1.0 is indicative of no increase over untreated sample (and is infact an indication of nil activity over control). Further, the meachnisms by which folic acid and gallated catechins are belived to interact are different (as described hereinabove) and therefore synergy is not to be interpreted by comparing the NO activity of the compositions with respect to the arithmetic sum of the individual contributions. The data in the table above indicate that the compositions indeed demonstrate synergy since the NO activity achievable by the combination between about a weight ratio of 1:1 to about 10:1, preferably 2.5:1 to 7.5:1 is not achievable at any concentration of the individual ingredients. The data in Table -1 above indicates that there is optimum activity between the gallated catechin (EGCG) and folic acid when the weight ratio is between 1:1 to 10:1 preferably between 2.5:1 to 7.5:1.

## Claims

1. 4 topical composition comprising
(i) 0.1 to 5 wt% folic acid;
(ii) 0.1 to 5 wt% a gallated catechin; and
(iii) a cosmetically acceptable base selected from the group consisting of cream, lotion, gel and emulsion.
wherein the weight ratio of gallated catechin: folic acid is in the range of 2.5:1 to 10:1

2. A composition as claimed in claim 1 wherein said weight ratio of gallated catechin: folic acid is in the range of 2.5:1 to 7.5:1.

3. A composition as claimed in claim 1 or 2 wherein said gallated catechin is epigallo catechin gallate (EGCG) or epicatechin gallate (ECG).

4. A composition as claimed in any one of the preceding claims wherein said gallated catechin is included in the composition as an extract of green tea.

5. A composition according to any one of claims 1 to 4 for use in improving microcirculation of skin.

## Patentansprüche

1. Topische Zusammensetzung, umfassend
(i) 0,1 bis 5 Gew.-% Folsäure,
(ii) 0,1 bis 5 Gew.-% eines Gallat-Catechins und
(iii) eine kosmetisch annehmbare Basis, ausgewählt aus der aus Creme, Lotion, Gel und Emulsion bestehenden Gruppe,
wobei das Gewichtsverhältnis von Gallat-Catechin:Folsäure in dem Bereich von 2,5:1 bis 10:1 liegt.

2. Zusammensetzung gemäß Anspruch 1, wobei das Gewichtsverhältnis von Gallat-Catechin:Folsäure in dem Bereich von 2,5:1 bis 7,5:1 liegt.

3. Zusammensetzung nach Anspruch 1 oder 2, wobei das Gallat-Catechin Epigallocatechingallat (EGCG) oder Epicatechingallat (ECG) ist.

4. Zusammensetzung nach irgendeinem der vorhergehenden Ansprüche, wobei das Gallat-Catechin als Extrakt von grünem Tee in der Zusammensetzung enthalten ist.

5. Zusammensetzung nach irgendeinem der Ansprüche 1 bis 4 für die Verwendung bei der Verbesserung der Mikrozirkulation von Haut.

## Revendications

1. Composition topique comprenant
(i) 0,1 à 5 % en masse d'acide folique ;
(ii) 0,1 à 5 % en masse d'une catéchine gallatée ; et
(iii) une base cosmétiquement acceptable choisie dans le groupe constitué d'une crème, d'une lotion, d'un gel et d'une émulsion,
dans laquelle le rapport massique de catéchine gallatée : acide folique se trouve dans l'intervalle de 2,5:1 à 10:1.

2. Composition selon la revendication 1, dans laquelle ledit rapport massique de catéchine gallatée : acide folique se trouve dans l'intervalle de 2,5:1 à 7,5:1.

3. Composition selon la revendication 1 ou 2, dans laquelle ladite catéchine gallatée est le gallate d'épigallocatéchine (EGCG) ou le gallate d'épicatéchine (ECG).

4. Composition selon l'une quelconque des revendications précédentes, dans laquelle ladite catéchine gallatée est incluse dans la composition comme un extrait de thé vert.

5. Composition selon l'une quelconque des revendications 1 à 4 pour une utilisation dans l'amélioration de la microcirculation de la peau.
